# EUROPEAN PATENT APPLICATION

(11) **EP 3 771 715 A1**
(43) Date of publication of application: **03.02.2021**
(21) Application number: 19460040.9
(22) Date of filing: 02.08.2019
(51) Int. Cl.: C07D 403/14, C07D 487/04, A61K 31/519

(54) **CRYSTALLINE FORMS OF BARICITINIB**

(71) Applicant: Zaklady Farmaceutyczne "Polpharma" S.A., 83-200 Starogard Gdanski (PL)
(72) Inventor: Nienaltowski, Tomasz, 83-200 Starogard Gdanski (PL); Rostankowski, Rafal, 83-200 Starogard Gdanski (PL); Kaminski, Jacek, 83-200 Starogard Gdanski (PL)
(74) Representative: Rechnio, Justyna Tatiana

(57) **Abstract**

The invention relates to novel crystalline forms of baricitinib i.e. PPH form I and PPH form II and processes for preparation thereof. It also refers to pharmaceutical compositions containing said crystalline forms and to a use of said crystalline forms or pharmaceutical compositions for the treatment of rheumatoid arthritis.

## Description

The invention relates to novel crystalline forms of baricitinib and processes for preparation thereof. It also refers to pharmaceutical compositions containing said crystalline forms and to a use of said crystalline forms or pharmaceutical compositions for the treatment of rheumatoid arthritis.

### Background art

Baricitinib, i.e. {1-(Ethylsulfonyl)-3-[4-(7H-pyrrolo[2,3-d]pyrimidin-4-yl)-1H-pyrazol-1-yl]-3-azetidinyl}acetonitrile, has the following structural formula (1):

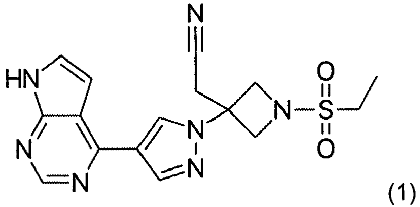

Baricitinib is an inhibitor of JAK1/JAK2 tyrosine kinase and is designated for treatment of rheumatoid arthritis.

WO2009/114512 A1 first discloses the process for preparation of baricitinib free base as off off-white solid (example 70).

WO2009/114512 A1 also discloses the process for preparation of baricitinib phosphate in a white crystalline form having a melting point of 187°C (example 71).

WO2009/114512 A1 further discloses the process preparation of baricitinib trifluoroacetate in an unknown form (example 1).

PCT publications number WO2015/166434, WO2016/141891, WO2019/003249 disclose various crystalline forms of baricitinib.

### Summary of the invention

The invention relates to novel crystalline forms of baricitinib i.e. PPH form I and PPH form II and processes for preparation thereof. It also refers to pharmaceutical compositions containing said crystalline forms and to a use of said crystalline forms or pharmaceutical compositions for the treatment of rheumatoid arthritis.

### List of figures

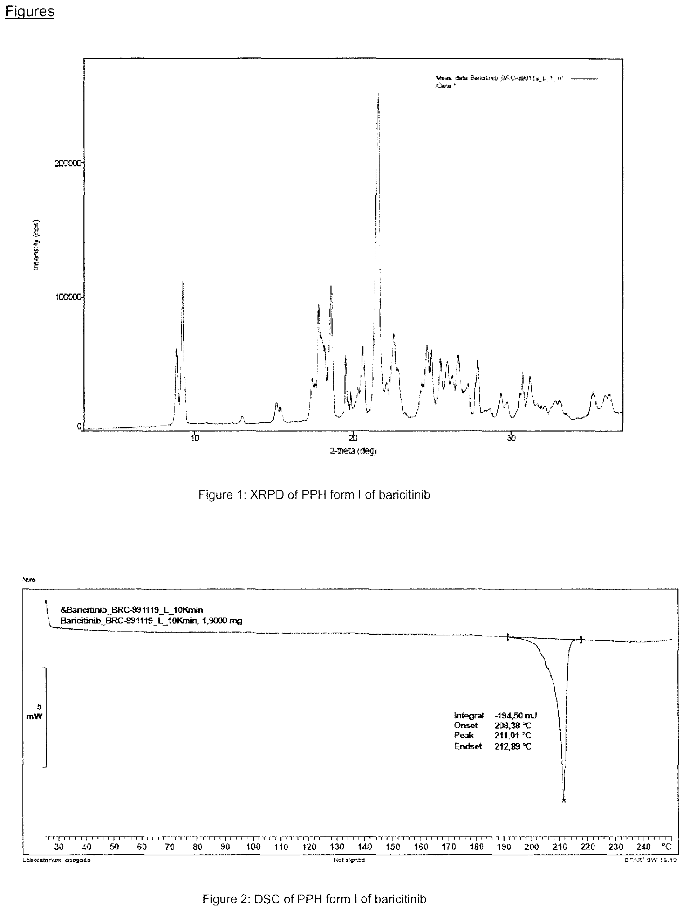

- Figure 1:: shows a representative XRPD pattern of PPH form I of baricitinib.
- Figure 2:: shows a representative DSC plot of PPH form I of baricitinib.

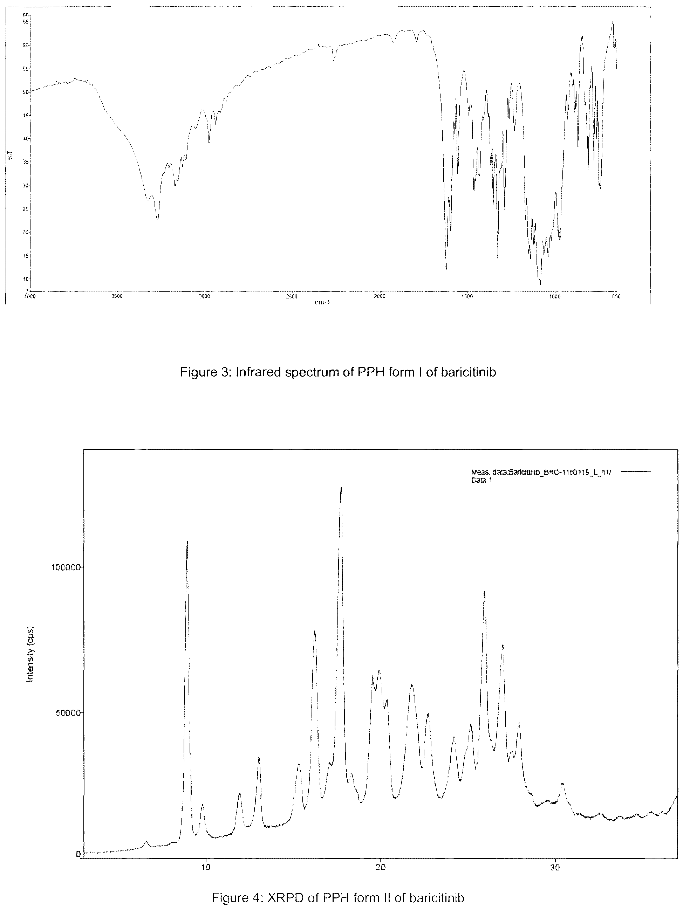

- Figure 3:: shows a representative IR spectrum of PPH form I of baricitinib
- Figure 4:: shows a representative XRPD pattern of PPH form II of baricitinib.

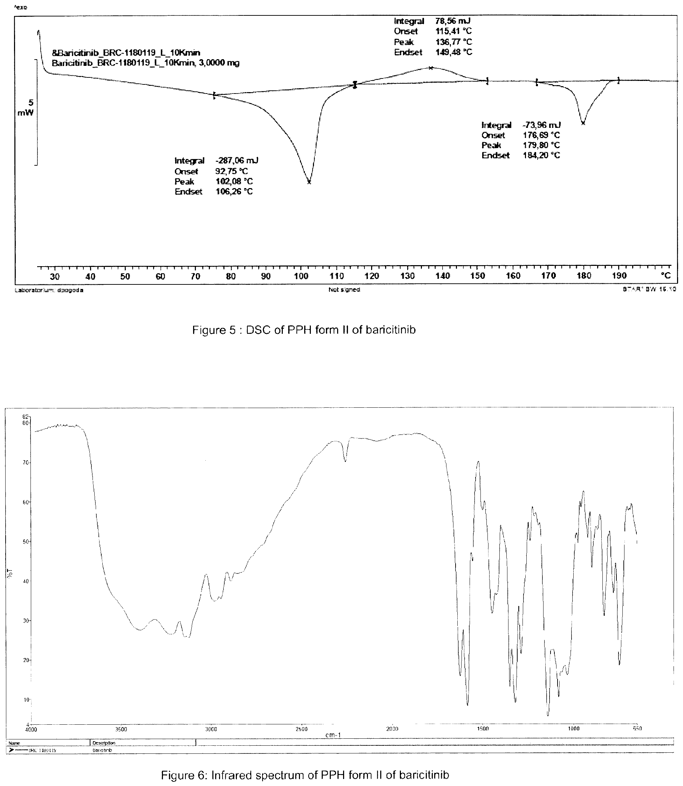

- Figure 5:: shows a representative DSC plot of PPH form II of baricitinib.
- Figure 6:: shows a representative IR spectrum of PPH form II of baricitinib

### Detailed description of the invention

The present invention relates to novel crystalline forms of baricitinib i.e. PPH form I and PPH form II and provides processes for preparation thereof.

It has unexpectedly been found that it is possible to provide new valuable crystalline forms of baricitinib, designated PPH form I and PPH form II herein. The crystalline forms described herein may have various benefits over the crystalline forms known from prior art.

In particular it has been found that crystalline PPH form I of baricitinib is substantially non-hygroscopic. In particular, crystalline PPH form I of baricitinib is morphologically homogeneous, contaminant-free, and stable.

Furthermore, crystalline PPH form II of baricitinib has been found, which is substantially non-hygroscopic. In particular, crystalline PPH form II of baricitinib is morphologically homogeneous, contaminant-free, and stable.

First aspect of the present invention is directed to the PPH form I of baricitinib.

The PPH form I of baricitinib of the present invention has an X-ray powder diffractogram with characteristic peaks at 2-theta angles of 8.8 degrees, 9.2 degrees, 15.2 degrees, 17.8 degrees, 18.6 degrees, 20.6 degrees, 21.5 degrees, 22.6 degrees, 24.7 degrees and 26.0 degrees, when using Cu-Ka radiation. Details of the X-ray powder diffraction measurement are provided, in the experimental part.

In more detail, the PPH form I of baricitinib of the present disclosure, has an X-ray powder diffractogram with peaks and relative intensities as shown in Table 1.

**Table 1: XRPD data of PPH form I of baricitinib.**

| No. | 2-theta(deg) | Height(cps) |
|---|---|---|
| 1 | 8.84 | 37317 |
| 2 | 9.24 | 69622 |
| 3 | 12.34 | 788 |
| 4 | 13.04 | 3969 |
| 5 | 14.63 | 953 |
| 6 | 15.21 | 10905 |
| 7 | 15.41 | 7591 |
| 8 | 17.42 | 20646 |
| 9 | 17.58 | 10135 |
| 10 | 17.82 | 55816 |
| 11 | 17.98 | 40052 |
| 12 | 18.23 | 12624 |
| 13 | 18.59 | 63769 |
| 14 | 19.52 | 38215 |
| 15 | 19.66 | 8490 |
| 16 | 19.83 | 13333 |
| 17 | 20.31 | 10400 |
| 18 | 20.62 | 33312 |
| 19 | 21.48 | 67875 |
| 20 | 21.60 | 105108 |
| 21 | 22.12 | 15351 |
| 22 | 22.33 | 9936 |
| 23 | 22.55 | 41388 |
| 24 | 22.79 | 21690 |
| 25 | 24.70 | 25070 |
| 26 | 24.91 | 17439 |
| 27 | 25.51 | 23202 |
| 28 | 25.95 | 23974 |
| 29 | 26.23 | 18008 |
| 30 | 26.59 | 23644 |
| 31 | 27.24 | 12457 |
| 32 | 27.67 | 20075 |
| 33 | 27.84 | 30120 |
| 34 | 29.34 | 10209 |
| 35 | 29.70 | 6780 |
| 36 | 30.57 | 11354 |
| 37 | 30.73 | 24037 |
| 38 | 31.20 | 18262 |
| 39 | 31.64 | 4872 |
| 40 | 32.15 | 4668 |
| 41 | 32.73 | 7050 |
| 42 | 33.07 | 8084 |
| 43 | 35.14 | 11855 |
| 44 | 35.94 | 9270 |
| 45 | 36.20 | 5522 |

According to another embodiment of the first aspect, the present invention is directed to PPH form I of baricitinib, wherein said PPH form I of baricitinib has a single endothermic peak in a differential scanning calorimetry plot with onset at 208±2°C.

According to another embodiment of the first aspect, the present invention is directed to PPH form I of baricitinib, wherein said PPH form I of baricitinib has an infrared spectrum comprising peaks at 3325 cm⁻¹, 3271 cm⁻¹, 1622 cm⁻¹, 1596 cm⁻¹, 1554 cm⁻¹, 1462cm¹, 1327 cm⁻¹, 1084 cm⁻¹, 869 cm⁻¹ and 739 cm⁻¹.

According to another embodiment of the first aspect, the present invention is directed to PPH form I of baricitinib, wherein said PPH form I of baricitinib is substantially chemically pure, i.e. it contains less than 1 area-%, or less than 0.5 area-%, preferably less than 0.1 area-%, of compounds other than baricitinib as determined by High Performance Liquid Chromatography (HPLC).

According to another embodiment of the first aspect, the present invention is directed to PPH form I of baricitinib, wherein said PPH form I of baricitinib is substantially polymorphically pure, and preferably contains less than 5 wt.-% of other crystalline, or amorphous forms of baricitinib thereof.

According to another embodiment of the first aspect, the present invention is directed to a process for preparing PPH form I of baricitinib as characterized above, wherein said process comprises the following steps:
a) deprotecting 2-(1-(ethylsulfonyl)-3-(4-(7-((2-(trimethylsilyl)ethoxy)methyl)-7H-pyrrolo[2,3-d]pyrymidin-4-yl)-1H-pyrazol-1-yl)azetidin-3-yl)acetonitrile with lithium tetrafluoroborate and aqueous ammonium hydroxide solution in a solvent selected from water, acetonitrile, methyl isobutyl ketone, heptane, cyclohexane, dimethylformamide, ethyl acetate, methanol, ethanol, isopropanol, dioxane, or mixture thereof, preferably in acetonitrile and water mixture,
b) adding ethanol to the reaction mixture obtained in step a) and heating to 50 - 60°C to obtain a suspension,
c) filtering the suspension obtained in step b) and distilling off solvent(s) from the filtrate to dryness,
d) crystallizing baricitinib PPH form I from a mixture of ethanol and water.

In a second aspect, the present invention relates to the PPH form II of baricitinib.

The PPH form II of baricitinib of the present invention has an X-ray powder diffractogram with characteristic peaks at 2-theta angles of 8.9 degrees, 11.9 degrees, 13.0 degrees, 16.2 degrees, 17.7 degrees, 19.5 degrees, 19.9 degrees, 21.8 degrees, 25.9 degrees and 26.9 degrees, when using Cu-Ka radiation. Further details regarding the X-ray powder diffraction measurement are provided herein below.

Furthermore, PPH form II of baricitinib of the present invention has an X-ray powder diffraction pattern with peaks and relative intensities as shown in Table 2.

**Table 2: XRPD of PPH form II of baricitinib**

| No. | 2-theta(deg) | Height(cps) |
|---|---|---|
| 1 | 6.56 | 1522 |
| 2 | 8.93 | 65416 |
| 3 | 9.83 | 7334 |
| 4 | 11.95 | 9641 |
| 5 | 13.04 | 17110 |
| 6 | 15.31 | 11408 |
| 7 | 16.23 | 39110 |
| 8 | 17.05 | 8123 |
| 9 | 17.73 | 65811 |
| 10 | 18.34 | 4109 |
| 11 | 19.55 | 26590 |
| 12 | 19.94 | 21584 |
| 13 | 20.37 | 22278 |
| 14 | 21.79 | 24963 |
| 15 | 22.70 | 17000 |
| 16 | 24.17 | 8047 |
| 17 | 24.88 | 3032 |
| 18 | 25.23 | 9462 |
| 19 | 25.96 | 41444 |
| 20 | 26.96 | 29428 |
| 21 | 27.44 | 5842 |
| 22 | 27.91 | 14035 |
| 23 | 30.46 | 5750 |
| 24 | 32.56 | 1050 |

According to another embodiment of the second aspect, the present invention is directed to provide PPH form II of baricitinib, wherein the said PPH form II of baricitinib has two broad endothermic peaks in a differential scanning calorimetry plot with onset at 92±2°C and 176±2°C.

According to another embodiment of the second aspect, the present invention is directed to PPH form II of baricitinib, wherein said PPH form II of baricitinib has infrared spectrum comprising peaks at 3392 cm⁻¹, 3224 cm⁻¹, 1626 cm⁻¹, 1585 cm⁻¹, 1447cm⁻¹, 1351 cm⁻¹, 1288 cm⁻¹, 1084 cm⁻¹, 899 cm⁻¹ and 748 cm⁻¹.

According to another embodiment of the second aspect, the present invention is directed to PPH form II of baricitinib, wherein said PPH form II of baricitinib is substantially chemically pure, i.e. it contains less than 1 area-%, or less than 0.5 area-%, preferably less than 0.1 area-%, of compounds other than baricitinib as determined by High Performance Liquid Chromatography (HPLC).

According to another embodiment of the second aspect, the present invention is directed to PPH form II of baricitinib, wherein said PPH form II of baricitinib is substantially polymorphically pure, and preferably contains less than 5 wt.-% of other crystalline, non-crystalline or amorphous forms of baricitinib.

According to another embodiment of the second aspect, the present invention is directed to a process for preparing PPH form II of baricitinib as characterised above, wherein said process comprises the following steps:
a) dissolving PPH form I of baricitinib in a mixture of water and one or more solvents selected from ethanol, acetone, acetonitrile, isopropanol or a mixture thereof, preferably in a mixture of water and ethanol.
b) slowly cool down the mixture of step a) for at least 2 hours at 0-5 °C,
c) optionally, seeding a mixture of step a) with crystals of PPH form II,
d) crystallizing the PPH form II of baricitinib.

Further object of the invention is a pharmaceutical composition comprising novel crystalline forms of baricitinib i.e. PPH form I and/or PPH form II.

In the preferred embodiment of the above, the pharmaceutical composition according to the present invention is formulated into a solid unit dosage form for oral administration, preferably into a tablet.

Preferably, the pharmaceutical composition and/or the solid unit dosage form as above, comprise at least on pharmaceutically acceptable excipient.

Exemplary pharmaceutically acceptable excipients which can be contemplated for use in the pharmaceutical composition of the present invention are described below.

Fillers are excipients which increase the volume of e.g. tablets. Examples of fillers are lactose, mannitol, cellulose and its derivatives such as microcrystalline cellulose, synthetic polymers, Ca-phosphate, inorganic calcium salts, maize starch, polyols and pregelatinized starch.

Binding agents are excipients which increase the adhesion of the active agents and excipients during granulation. Examples of binding agents are: sugars, such as sorbitol, glucose, fructose, disaccharides as saccharose, polysaccharides; acacia gum; cellulose derivatives, such as soluble celluloses like methylcellulose, hydroxypropyl methylcellulose and hydroxypropyl cellulose; tragacanth; polyvinylpyrrolidone, such as N-vinylpyrrolidone or polyvinylpyrrolidone-vinyl acetate copolymer; sodium alginate and alginate derivatives and gelatin.

Disintegrants are excipients which can take up water and swell and thus improve disintegration of a tablet or granules. Examples of disintegrants are: croscarmellose sodium; starch (paste and pre-gelatinized), such as sodium starch glycolate, croscarmellose sodium and low substituted hydroxypropyl cellulose, methacrylic acid polymer with divinylbenzene, potassium salt, maltodextrin; and crospovidone.

Lubricants are excipients which reduce the friction between excipients and compression tooling surfaces. Examples of lubricants are magnesium stearate, magnesium fumarate, fumaric acid, and sodium stearyl fumarate.

A polymer, or a plurality of polymers may be used to coat the solid dosage form, if necessary, and they include cellulose derivatives, e.g. hydroxypropyl methylcellulose, polyvinylpyrrolidones, polyethylene glycols, polyvinyl alcohols, acrylates, such as polymethacrylate, cyclodextrins and copolymers and derivatives thereof, including for example polyvinylpyrolidine-vinylacetate. In some preferred embodiments the polymer or the plurality of polymers are pH dependent enteric polymers. Such polymers include cellulose derivatives, e.g. cellulose acetate phthalate, hydroxypropyl methyl cellulose phthalates, hydroxypropyl methyl acetate succinate, hydroxypropyl methyl cellulose acetate, carboxymethylcellulose or a salt thereof, e.g. the sodium salt, cellulose acetate trimellitate, hydroxypropyl cellulose acetate phthalate, or polymethyl acrylates, e.g. Eudragit®S.

Surfactants include but are not limited to, sorbitan fatty esters, polyoxymethylene sorbit esters, sodium lauryl sulfate, sodium docedylbenzenesulfate, dioctyl sodium sulfosuccinate, sodium stearate, EDTA or vitamin E or tocopherol derivatives.

Other excipients are known in the art and can be chosen by a skilled person depending on their function. Reference may be made in this context to Handbook of Pharmaceutical Excipients, ed. by R.C. Rowe et al, 6th edition, Pharmaceutical Press(2009).

Formulating the said crystalline form into a pharmaceutical dosage form may be carried out by applying techniques and excipients known in the art. Reference is made herein for example to Pharmaceutical Manufacturing Handbook; Production and Processes, ed. S.C. Gad; John Wiley and Sons, (2008).

Another object of the present invention is the novel crystalline form of baricitinib i.e. PPH form I and/or PPH form II, or the pharmaceutical composition comprising any of the said crystalline forms for the treatment of rheumatoid arthritis.

### Experimental part

### Measuring instruments, conditions and protocols:

Within the meaning of the present invention, the term "room temperature" as used herein is understood to mean temperatures of about 15°C to about 25°C.

### HPLC

Purity and assay analysis was carried out using High Performance Liquid Chromatograph equipped with PDA detector. The analysis was conducted using Octadecyl silane (ODA or C18) chemically bonded to porous silica column, at a temperature of 40°C. The mobile phases was acetonitrile and 0.1% (v/v) ortho-phosphoric acid in water used in gradient.

### KF

Water content in samples of baricitinib was determined using Mettler Toledo V30 Karl Fisher Titrator. The analysis was carried out using acetonitrile : methanol (50:50) solution and the Combi Titrant 2 Merck titrant.

### XRPD

Powder X-ray diffraction was performed with a Rigaku MiniFlex 600 diffractometer with Bragg-Brentano geometry CuKa radiation and D/teX Ultra Detector. The powder X-ray diffraction patterns were recorded at a tube voltage of 40kV and a tube current of 15 mA, applying a step size of 0.01° and speed 6.0°/min with the angular range 3-36° at ambient conditions.

Herein, the X-ray powder diffractogram peaks are described by their 2theta angles with a tolerance of "±0.2". In the context of the present invention, it is also possible to use a smaller tolerance of "±0.1".

### DSC

DSC analysis was carried out using DSC 1 Mettler Toledo Stare thermal analysis system in the temperature range of 30-250°C with the gradient of 10°C/min. Aluminum crucible with the capacity of 40µl were used. Nitrogen was used as carrier gas with the flow rate of 40ml/min. The accuracy of the measurements is ±2 °C.

### IR measurements

IR analysis were performed on FT-IR Thermo Nicolet Avatar 370 spectrometer in ATR method and PIKE TECHNOLOGIES Smart MIRacle TM single Reflection HATR module. The accuracy of the measurements is ±2 cm⁻¹.

### Crystalline Stability

A test for crystalline stability against humidity can be performed as follows: The sample is exposed to a relative humidity of 75% (±5%) at 40°C (±2°C) for 4 weeks. The expression "crystalline stability" means that no conversion into another crystalline form occurs, as determined by XRPD.

The equilibrium relative humidity of a sample is measured by determining the relative humidity in % in the air above a test sample, after establishment of a humidity equilibrium in a closed/open system at a constant temperature.

The following examples describe the present invention in detail, but are not to be construed to limit the present invention.

### Example 1 - Preparation of 4-(1H-pyrazol-4-yl)-7-((2-(trimethylsilil)ethoxy)methyl)-7H-pyrrolo[2,3-d]pyrimidine (6)

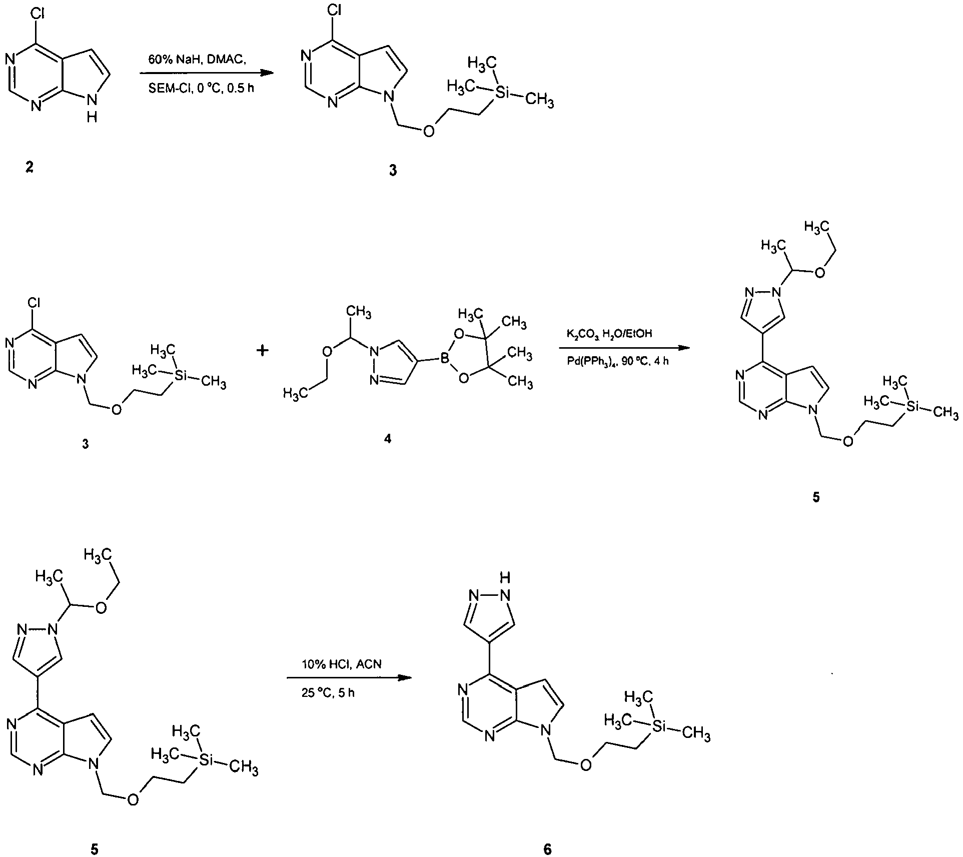

To a mixture of sodium hydride (7.15 g, 0.1797 mol, 1.1 eq) in N,N,-dimethylacetamide (100 ml), 4-chloro-7H -pyrrolo[2,3-d]pyrimidine (25 g, 0.1628 mol, 1 eq) in N,N,-dimethylacetamide (150 ml) is added under stirring while maintaining the temperature of reaction mass at 0-5°C. The resulting reaction mass is stirred for 30 minutes at 0-5°C. SEM chloride (trimethylsilyl ethoxymethyl chloride) (30 g, 0.1797 mol, 1.1 eq) is then added dropwise to the reaction mixture while maintaining the temperature about 0-5°C. The reaction mixture is stirred at 0-5°C for 1-2 hours. After the completion of the reaction, the reaction mass is quenched with water (50 ml). The reaction mixture is diluted with additional water (200 ml) and is extracted with ethyl acetate (250 ml). The aqueous layer is extracted twice with ethyl acetate (250 ml). The combined organic layers are washed with brine (125 ml) and with water (250 ml). The organic layer is concentrated at 40°C under vacuum to obtain 4-chloro-7-[2-(trimethylsilyl)ethoxy]methyl-7H-pyrrolo[2,3-d]pyrimidine (3).

To 4-chloro-7-[2-(trimethylsilyl)ethoxy]methyl-7H-pyrrolo[2,3-d]pyrimidine (3) a mixture of solvent [water (115.5 ml) and ethanol (347 ml)], potassium carbonate (50.6 g, 0.3663 mol, 2.25 eq), 1-(1-ethoxyethyl)-4-(4,4,5,5-tetramethyl-1,3,2-dioxaborolan-2yl)-1H-pyrazole (45.5 g, 0.1710 mol, 1.05 eq) is added at room temperature. Resulting reaction mixture is degassed with nitrogen for 20 min and terakis(triphenylphosphine)palladium(0) (0.38 g, 0.000329 mol, 0.002 eq) is added as catalyst at room temperature. The reaction mixture is kept under gentle reflux at 85-90°C for 2 hrs. After the completion of reaction, the reaction mixture is cooled to room temperature. The suspension is filtered and washed with ethyl acetate (225 ml). Layers are separated and the organic layer is concentrated under reduced pressure at 45°C to obtain 4-(1-(1-ethoxyethyl)-1H-pyrazol-4-yl)-7-((2-(trimethylsilyl)ethoxy)methyl)-7H-pyrrolo[2,3-d]pyrimidine (5).

To 4-(1-(1-ethoxyethyl)-1H-pyrazol-4-yl)-7-((2-(trimethylsilyl)ethoxy)methyl)-7H-pyrrolo[2,3-d]pyrimidine(5) acetonitrile (50 ml) and water (135 ml) are added. Reaction mixture is heated to 35-45°C and 10% aqueous HCl solution is added at room temperature to adjust the pH below 0.5. Resulting reaction mixture is stirred at 35-45°C for 2 h. After the completion of reaction, reaction mixture is cooled to 20-30°C. The solid is collected by filtration, washed with water (250 ml) and dried at reduced pressure at 50-60°C to obtain [4-(1H-pyrazol-4-yl)-7-((2-(trimethylsilyl)ethoxy)methyl)-7H-pyrrolo[2,3-d]pyrimidine (6).
Yield: 41.6 - 45.7 g (80-90 %)
HPLC Purity: 98-99%

### Example 2 -Preparation of 2-(1-(ethylsulfonyl)-3-(4-(7-((2-(trimethylsilyl)ethoxy)methyl)-7H-pyrrolo[2,3-d]pyrymidin-4-yl)-1H-pyrazol-1-yl)azetidin-3-yl)acetonitrile (8)

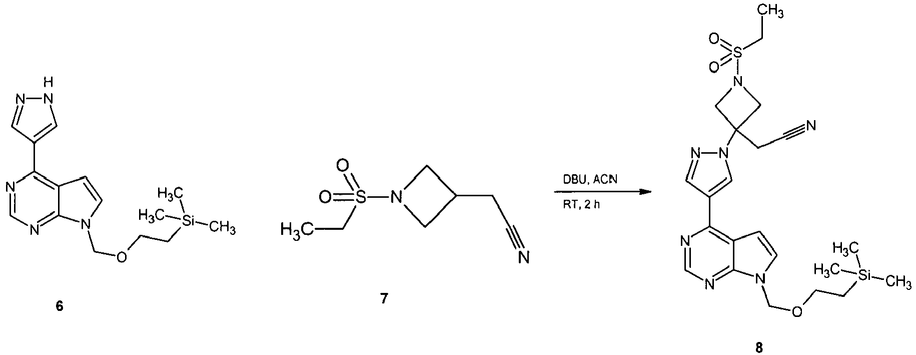

[4-(1H-pyrazol-4-yl)-7-((2-(trimethylsilyl)ethoxy)methyl)-7H-pyrrolo[2,3-d]pyrimidine(6) (40.0 g, 0.1268 mol, 1.0 eq) and [1-(ethylsulfonyl)azetidin-3-yl idene]acetonitrile (7) (22.4 g, 0.1205 mol, 0.95 eq) are added to a reactor containing acetonitrile (300 ml). DBU (19.3 g,0.1268 mol, 1.0 eq) is slowly added at 20-30°C. Reaction mass is stirred for 2 h at 20-30°C. After completion of the reaction, water (1000 ml) is added into the reaction mass and stirred for 2 hours at room temperature. The solid is filtered, washed with water (400 ml) and methyl tert-butyl ether (200 ml), and dried at 50°C in air dryer for 4-5 hrs. to obtain 2-(1-(ethylsulfonyl)-3-(4-(7-((2-(trimethylsilyl)ethoxy)methyl)-7H-pyrrolo[2,3-d]pyrymidin-4-yl)-1H-pyrazol-1-yl)azetidin-3-yl)acetonitrile (8).
Yield: 48.9 - 54.1 g (76 - 86%)
HPLC Purity: 98-99%

### Example 3 -Preparation of PPH form I of baricitinib

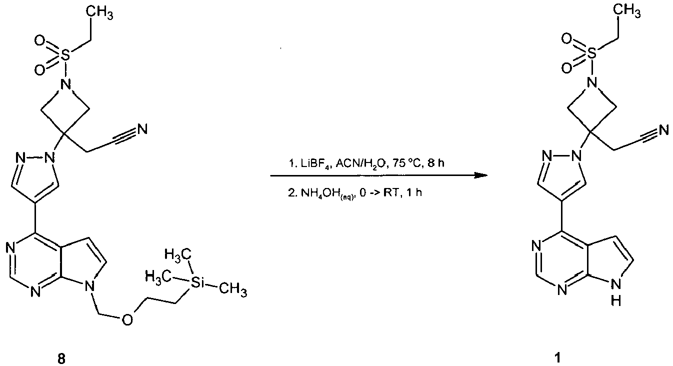

To a solution of 2-(1-(ethylsulfonyl)-3-(4-(7-((2-(trimethylsilyl)ethoxy)methyl)-7H-pyrrolo[2,3-d]pyrymidin-4-yl)-1H-pyrazol-1-yl)azetidin-3-yl)acetonitrile (8) (50 g, 0.0996 mol, 1.0 eq) in acetonitrile (450 ml) and water (45 ml), lithium tetrafluoroborate (102.7 g, 1.0963 mol, 11 eq) is added at room temperature (20-30°C). The reaction mixture is heated to 80-90°C and stirred for 12 hour. After the completion of reaction, the reaction mixture is cooled to 0-10°C, solution of ammonium hydroxide 25% is added slowly into the reaction mixture to adjust the pH to 9-10. The reaction mixture is then stirred for 1 hour at room temperature. Next, ethanol (500 ml) is added to reaction mixture, followed by heating at 50-60°C. The reaction mixture is stirred for 1 h at 50-60°C, filtered and washed with ethanol (150 ml). Filtrate is distilled out under reduced pressure till dryness, EtOH: water (3:1) (750 ml) mixture is added and the reaction mixture is heated to 50°C and stirred until complete dissolution. Subsequently the reaction mixture is gradually cooled to 0-10°C and stirred for 2 h at 0-10°C. Solid is filtered off and washed with water (250 ml). Obtained solid is dried at 45°C under vacuum to obtain PPH form I of baricitinib.
Yield: 29.9 - 33.1 g (80 - 90%)
HPLC Purity: 99.5-100%
K.F. - 0.30 %

### Example 4 -Preparation of PPH form II of baricitinib

PPH form I of baricitinib (30 g, 0.0807 mol, 1.0 eq) is suspended in ethanol/water mixture 3:1(450 ml) and the reaction mass is heated to 70-80°C to obtain clear solution. Activated carbon (3 g) is added followed by stirring for 1 h at 70-80°C. Suspension is filtered and washed with ethanol/water mixture 3:1 (60 ml). Filtrate is extracted with n-heptane , the n-heptane layer is discarded and the reaction mixture is gradually cooled down to 0-5°C within 4 h and stirred for another 2 h. Solid is filtered, washed with water (45 ml) and the obtained solid is dried at 45°C under vacuum for 8-10 hrs. to provide PPH form II of baricitinib.
Yield: 24.3 - 27.3 g (81 - 91%)
Purity: 99.5-100%
K.F. - 6.61 %

### Example 5 -Preparation of PPH form II of baricitinib

PPH form I of baricitinib (30 g, 0.0807 mol, 1.0 eq) is suspended in ethanol/water mixture 3:1 (450 ml) and reaction mass is heated to 70-80°C to obtain clear solution. Cool the reaction mixture to 0-5°C within 4 h and stirred for another 2 h. Solid is filtered, washed with water (45 ml) and obtained solid is dried at 45°C under vacuum 8-10 hrs. to provide PPH form II of baricitinib.
Yield: 25.10 - 28.30 g (83.6 - 94.3%)
Purity: 99.5-100%

### Example 6 -Preparation of amorphous form of baricitinib

PPH form II of baricitinib obtained in example 4 (20 g) is dried at 100-120°C in an air dryer to obtain amorphous form of baricitinib as confirmed by XRPD analysis.
Yield : 98%

### Example 7 -Crystalline stability study of novel crystalline forms of baricitinib

A test for crystalline stability against humidity has been performed as follows: The sample is exposed to a relative humidity of 75% (±5%) at 40°C(±2°C) for 4 weeks. The expression "crystalline stability" means that no conversion into another crystalline form occurs, as determined by XRPD.

The equilibrium relative humidity of a sample is measured by determining the relative humidity in % in the air above a test sample, after establishment of a humidity equilibrium in a closed/open system at a constant temperature.

The results are summarized in the Table :

**Table 3: Crystalline stability of PPH form I and PPH form II of baricitinib**

| Crystalline Forms | XRPD (crystallinity) | Crystalline stability study 40°C, 75 ± 5% RH After 1 week | Crystalline stability study 40°C, 75 ± 5% RH After 4 weeks |
|---|---|---|---|
| PPH form I | Good | Stable | Stable |
| PPH form II (Example 4) | Good | Stable | Stable |

XRPD results show that the PPH form I and PPH form II of baricitinib remain stable after 4 weeks at 40°C, 75 ± 5% relative humidity.

### Example 8- Chemical stability study of new crystalline forms of baricitinib

Chemical stability study of the new crystalline forms of baricitinib i.e. PPH form I and PPH form II was performed under accelerated ICH storage conditions (40°C, 75±5% relative humidity). Samples were taken periodically (0 (start), after 1, 2, and 4 weeks) and analyzed by HPLC.

**Table 4 : HPLC purity of PPH form I of baricitinib**

| | Name | RT | RRT | Height | Res | As | EP s/n | Area | % Area |
|---|---|---|---|---|---|---|---|---|---|
| 1 | Ethyl Acetate | 9.603 | | | | | | | |
| 2 | BRC-6 | 10.888 | | | | | | | |
| 3 | BRC | 11.674 | 1.000 | 2767837 | | 0.9 | 91326.8 | 16879660 | 99.96 |
| 4 | BRC-8 | 12.791 | | | | | | | |
| 5 | RRT∼ 1.306 | 15.245 | 1.306 | 339 | 23.0 | 1.0 | 10.2 | 1746 | 0.01 |
| 6 | BRC-1 | 16.376 | | | | | | | |
| 7 | RRT∼ 1.411 | 16.466 | 1.411 | 872 | 7.8 | | 27.8 | 5520 | 0.03 |
| 8 | BRC-5 | 27.929 | | | | | | | |
| 9 | BRC-7 | 34.151 | | | | | | | |
| 10 | BRC-4 (int) | 35.544 | | | | | | | |
| 11 | BRC-2 | 37.358 | | | | | | | |

**Table 5: Chemical stability of PPH form I of baricitinib at 40°C, 75 ± 5% RH - summary of HPLC analysis**

| Time (Week) | Impurities (HPLC % a/a) | |
|---|---|---|
| | RRT 1.30 | RRT 1.41 |
| 0 | 0.01 | 0.03 |
| 1 | 0.01 | 0.03 |
| 2 | 0.01 | 0.03 |
| 4 | 0.01 | 0.03 |

HPLC results show that the PPH form I of baricitinib remains stable during 0,1, 2, and 4 weeks at 40°C, 75 ± 5% relative humidity.

**Table 6: HPLC Purity of PPH form II of baricitinib**

| | Name | RT | RRT | Height | Res | As | EP s/n | Area | % Area |
|---|---|---|---|---|---|---|---|---|---|
| 1 | Ethyl Acetate | 9.603 | | | | | | | |
| 2 | BRC-6 | 10.888 | | | | | | | |
| 3 | BRC | 11.701 | 1.000 | 2874824 | | 0.9 | 79111.6 | 17701201 | 99.65 |
| 4 | BRC-8 | 12.791 | | | | | | | |
| 5 | BRC-1 | 16.376 | | | | | | | |
| 6 | RRT∼ 1.408 | 16.479 | 1.408 | 876 | 29.6 | | 23.1 | 5260 | 0.03 |
| 7 | RRT∼ 1.702 | 19.915 | 1.702 | 4778 | 21.3 | 1.1 | 130.5 | 29874 | 0.17 |
| 8 | RRT∼ 2.204 | 25.788 | 2.204 | 4162 | 35.4 | 1.0 | 113.5 | 26258 | 0.15 |
| 9 | BRC-5 | 27.929 | | | | | | | |
| 10 | BRC-7 | 34.151 | | | | | | | |
| 11 | BRC-4 (int) | 35.544 | | | | | | | |
| 12 | BRC-2 | 37.358 | | | | | | | |

**Table 7: Chemical stability of PPH form II of baricitinib at 40°C, 75 ± 5% RH - summary of HPLC analysis**

| Time (Week) | Impurities (HPLC % a/a) | | |
|---|---|---|---|
| | RRT 1.40 | RRT 1.70 | RRT 2.20 |
| 0 | 0.03 | 0.17 | 0.15 |
| 1 | 0.03 | 0.17 | 0.15 |
| 2 | 0.03 | 0.17 | 0.15 |
| 4 | 0.03 | 0.17 | 0.15 |

HPLC results show that the PPH form II of baricitinib remains stable during 0,1, 2, and 4 weeks at 40°C, 75 ± 5% relative humidity.

## Claims

1. Crystalline PPH form I of baricitinib having an X-ray powder diffractogram with characteristic peaks at 2-theta angles±0.2 2-theta degree of 8.8 degrees, 9.2 degrees, 15.2 degrees, 17.8 degrees, 18.6 degrees, 20.6 degrees, 21.5 degrees, 22.6 degrees, 24.7 degrees and 26.0 degrees when using Cu-Ka radiation.

2. The crystalline PPH form I of baricitinib of claim 1, having a single endothermic peak in a differential scanning calorimetry plot with onset at 208±2°C.

3. The crystalline PPH form I of baricitinib of claim 1, having an infrared spectrum comprising peaks at 3325 cm⁻¹, 3271 cm⁻¹, 1622 cm⁻¹, 1596 cm⁻¹, 1554 cm⁻¹, 1462 cm⁻¹, 1327 cm⁻¹, 1084 cm⁻¹, 869 cm⁻¹ and 739 cm⁻¹.

4. A process for preparing crystalline PPH form I of baricitinib according to any of claims 1 to 3, wherein said process comprises the following steps:
a) deprotecting 2-(1-(ethylsulfonyl)-3-(4-(7-((2-(trimethylsilyl)ethoxy)methyl)-7H-pyrrolo[2,3-d]pyrymidin-4-yl)-1H-pyrazol-1-yl)azetidin-3-yl)acetonitrile with lithium tetrafluoroborate and aqueous ammonium hydroxide solution in a solvent selected from water, acetonitrile, methyl isobutyl ketone, heptane, cyclohexane, dimethylformamide, ethyl acetate, methanol, ethanol, isopropanol, dioxane, or mixture thereof, preferably in acetonitrile and water mixture,
b) adding ethanol to the reaction mixture obtained in step a) and heating to 50-60°C to obtain a suspension,
c) filtering the suspension obtained in step b) and distilling of solvent(s) from the filtrate to dryness,
d) crystallizing baricitinib PPH form I from a mixture of ethanol and water.

5. Crystalline PPH form II of baricitinib having an X-ray powder diffractogram peaks at 2-theta angles ±0.2 2-theta degree of 8.9 degrees, 11.9 degrees, 13.0 degrees, 16.22 degrees, 17.7 degrees, 19.5 degrees, 19.9 degrees, 21.8 degrees, 25.9 degrees and 26.9 degrees, when using Cu-Ka radiation.

6. The crystalline PPH form II of baricitinib of claim 5, having two broad endothermic peaks in a differential scanning calorimetry plot with onset at 92±2°C and 176 ±2°C.

7. The crystalline PPH form II of baricitinib of claim 5 or 6, having an infrared spectrum comprising peaks at 3392 cm⁻¹, 3224 cm⁻¹, 1626 cm⁻¹, 1585 cm⁻¹, 1447 cm⁻¹, 1351 cm⁻¹, 1288 cm⁻¹, 1084 cm⁻¹, 899 cm⁻¹, 748 cm⁻¹.

8. A process for preparing crystalline PPH form II of baricitinib according to any of claims 5 to 7, wherein said process comprises the following steps:
a) dissolving PPH form I of baricitinib in a mixture of water and one or more solvents selected from ethanol, acetone, acetonitrile, isopropanol or a mixture thereof, preferably in a mixture of water and ethanol,
b) slowly cool down the mixture of step a) for at least 2 hours at 0-5°C,
c) optionally, seeding a mixture of step a) with crystals of PPH form II,
d) crystallizing the PPH form II of baricitinib.

9. Pharmaceutical composition or dosage form comprising a crystalline form of any of claims 1-3 and/or 5-7.

10. The pharmaceutical composition or dosage form of claim 9, comprising at least one pharmaceutically acceptable excipient.

11. Pharmaceutical composition or dosage form of claim 9 or 10 for use in a method of treating rheumatoid arthritis.
